# EUROPÄISCHE PATENTANMELDUNG

(11) **EP 3 391 802 A1**
(43) Veröffentlichungstag der Anmeldung: **24.10.2018**
(21) Anmeldenummer: 18167322.9
(22) Anmeldetag: 13.04.2018
(51) Int. Cl.: A61B 1/00

(54) **ENDOSKOPISCHE SONDE**

(30) Priorität: 19.04.2017 DE 102017108272
(71) Anmelder: Carl Zeiss Meditec AG, 07745 Jena (DE)
(72) Erfinder: FANENBRUCK, Martin, 73447 Oberkochen (DE); JESS, Helge, 73447 Oberkochen (DE); GUCKLER, Roland, 89081 Ulm (DE)
(74) Vertreter: PATERIS Patentanwälte PartmbB

(57) **Zusammenfassung**

Es wird eine endoskopische Sonde zur Verfügung gestellt. Diese umfasst:
- einen Handgriff mit einem eine Vorderende, einem Hinterende mit einer Hinterendenachse und einem sich zwischen dem Vorderende und dem Hinterende erstreckenden geraden Griffabschnitt mit einer Griffabschnittsachse;
- einen sich vom Vorderende des Handgriffs aus erstreckenden Schaft mit einer Schaftachse und
- ein aus dem Hinterende austretendes Kabel, wobei
- die Schaftachse, die Hinterendenachse und die Griffabschnittsachse innerhalb einer gemeinsamen Ebene liegen,
- die Schaftachse mit der Griffabschnittsachse einen Winkel im Bereich von 100 bis 140 Grad einschließt,
- die Griffabschnittsachse mit der Hinterendenachse einen Winkel im Bereich von 110 bis 130 Grad einschließt,
- die Schaftachse mit der Hinterendenachse einen Winkel im Bereich von 30 bis 90 Grad einschließt.

## Beschreibung

Die vorliegende Erfindung betrifft eine endoskopische Sonde mit einem Schaft und einem Handgriff, welcher ein Vorderende, ein Hinterende sowie einen sich zwischen dem und dem Hinterende erstreckenden Griffabschnitt umfasst.

In der Mikrochirurgie, wie etwa in der Neurochirurgie, besteht beispielsweise nach dem Entfernen eines tief liegenden Tumors oder der Behandlung eines Aneurysmas regelmäßig Bedarf an Orte zu schauen, die außerhalb der Sichtlinie eines Operationsmikroskops liegen. Zu diesem Zweck kommen dann zusätzlich zum Operationsmikroskop endoskopische Sonden zum Einsatz, wie sie beispielsweise in DE 10 2015 004 546 A1, US 7,162,292 B2 und in US 2011/0178395 A1 beschrieben sind.

Endoskopische Sonden weisen typischerweise einen Handgriff auf, von dessen einen Ende sich ein Schaft erstreckt und von dessen anderem Ende ein Kabel abgeht. Dabei besteht eine Herausforderung darin, die endoskopische Sonde so zu gestalten, dass sie einerseits eine hohe Ergonomie aufweist und andererseits eine Kollision der endoskopischen Sonde mit einem zusammen mit ihr verwendeten Operationsmikroskop zuverlässig vermieden werden kann.

Aufgabe der vorliegenden Erfindung ist es eine endoskopische Sonde zur Verfügung zu stellen, die eine hohe Ergonomie aufweist und mit der sich eine Kollision mit einem zusammen mit ihr verwendeten Operationsmikroskop zuverlässig vermeiden lässt.

Diese Aufgabe wird durch eine endoskopische Sonde nach Anspruch 1 gelöst. Die abhängigen Ansprüche enthalten vorteilhafte Ausgestaltungen der Erfindung.

Eine erfindungsgemäße endoskopische Sonde umfasst einen Handgriff mit einem Vorderende, einem Hinterende mit einer Hinterendenachse und einem sich zwischen dem Vorderende und dem Hinterende erstreckenden geraden Griffabschnitt mit einer Griffabschnittsachse. Außerdem umfasst sie einen sich vom Vorderende des Handgriffs aus erstreckenden Schaft mit einer Schaftachse und, wenn das Hinterende als Kabelabgangsende ausgebildet ist, ein aus dem Hinterende austretendes Kabel. Dabei sind die Achsen jeweils durch die Verbindungslinie zwischen den Mittelpunkten der Querschnitte an den Enden des jeweiligen Abschnitts bzw. des Schaftes gegeben. In der erfindungsgemäßen endoskopischen Sonde liegen die Schaftachse, die Hinterendenachse und die Griffabschnittsachse innerhalb einer Ebene. Die Schaftachse schließt mit der Griffabschnittsachse einen Winkel im Bereich von 100 bis 140 Grad, insbesondere im Bereich von 110 bis 130 Grad und vorzugsweise im Bereich von 115 bis 125 Grad, ein. Die Griffabschnittsachse schließt mit der Hinterendenachse einen Winkel im Bereich von 110 bis 130 Grad, insbesondere im Bereich von 115 bis 125 Grad, ein, und die Schaftachse schließt mit der Hinterendenachse einen Winkel im Bereich von 30 bis 90 Grad ein. Dabei kann der Winkel, den die Schaftachse mit der Griffabschnittsachse einschließt, insbesondere denselben Betrag besitzen wie der Winkel, den die Griffabschnittsachse mit der Hinterendenachse einschließt. Falls vorhanden, tritt das Kabel in einem Winkel von nicht mehr als maximal 20 Grad, vorzugsweise von nicht mehr als 10 Grund zur Hinterendenachse aus dem Hinterende aus. Insbesondere kann das Kabel parallel zur Hinterendenachse aus dem Hinterende austreten. Inbesondere im Falle einer kabellosen endoskopischen Sonde kann das Hinterende eine Antenne und/oder einen Akkumulator enthalten. Aber auch im Falle einer endoskopischen Sonde mit Kabel kann das Hinterende eine Antenne und/oder einen Akkumulator enthalten.

Die Abwinklung der erfindungsgemäßen endoskopischen Sonde gemäß den angegebenen Winkeln ermöglicht es, die Gesamtlänge in Richtung der Griffabschnittsachse gering zu halten sowie das Risiko einer Kollision des Handgriffs oder des Hinterendes bzw. Kabelabgangs mit einem zeitgleich eingesetzten Operationsmikroskop zu minimieren, wobei gleichzeitig eine hohe Ergonomie erreicht wird. Zwar wäre im Sinne der Minimierung des Risikos einer Kollision ein Winkel zwischen der Schaftachse und der Handgriffachse von 90 Grad optimal, jedoch hat sich gezeigt, dass sich bei einem derartigen Winkel keine gute Handhabbarkeit in der vorwiegend einhändigen Benutzung der endoskopischen Sonde ergibt. Insbesondere das Drehen im Situs sowie das sichere Einführen der Sonde werden durch einen Winkel von 90 Grad beeinträchtigt. Der erfindungsgemäße Winkel zwischen der Schaftachse und der Handgriffachse, d.h. der Griffabschnittsachse, im Bereich von 100 bis 140 Grad, insbesondere im Bereich von 110 bis 130 Grad und vorzugsweise im Bereich von 115 bis 125 Grad ermöglicht ein unkompliziertes Drehen des Schaftes im Situs sowie das sichere Einführen in den Situs ohne gleichzeitig das Risiko einer Kollision mit dem gleichzeitig verwendeten Operationsmikroskop wesentlich zu erhöhen. Dadurch, dass die Hinterendenachse ebenfalls abgewinkelt ist, und zwar in einem Bereich zwischen 110 und 130 Grad, insbesondere zwischen 115 und 125 Grad, kann vermieden werden, dass das Hinterende bzw. das Kabel beim Drehen des Schaftes im Situs oder beim Einführen des Schaftes mit dem Operationsmikroskop kollidiert. Gleichzeitig kann durch den gewählten Winkel der Hinterendenachse das Kabel so über den Handrücken abgeführt werden, dass es den Anwender beim Drehen der Sonde im Situs nicht unnötig behindert. Die angegebenen Winkel kennzeichnen daher eine besonders vorteilhafte Geometrie der endoskopischen Sonde.

In der erfindungsgemäßen endoskopischen Sonde kann der Schaft eine Länge im Bereich von 50 bis 180 mm, insbesondere von 90 bis 130 mm aufweisen. Diese ist einerseits lang genug, um in mikrochirurgischen Eingriffen zum Einsatz zu kommen und andererseits kurz genug, damit eine Kollision des Handgriffs mit dem Operationsmikroskop beim Handhaben der endoskopischen Sonde vermieden werden kann.

Für den Schaft kommen Durchmesser im Bereich von 3,0 bis 4,1 mm, insbesondere im Bereich von 3,4 bis 3,8 mm in Frage. Dabei kann der Schaft zumindest in einem distalen Abschnitt eine Wandstärke im Bereich von 0,3 bis 0,5 mm, insbesondere im Bereich von 0,35 bis 0,45 mm aufweisen. An einem sich an das Vorderende des Handgriffs anschließenden proximalen Abschnitt des Schaftes kann dieser eine vergrößerte Wandstärke im Bereich von 1,3 bis 1,5 mm, insbesondere im Bereich von 1,35 bis 1,45 mm, aufweisen. Dabei kann der proximale Abschnitt eine Länge im Bereich von 10 bis 20 mm, insbesondere im Bereich von 13 bis 17 mm besitzen. Ein Schaft mit den genannten Abmessungen ist einerseits klein genug, um mit einem möglichst geringen chirurgischen Eingriff auskommen zu können und andererseits stabil genug, um ein Beschädigen des Schaftes während eines operativen Eingriffs zu vermeiden. Zudem ist der lichte Schaftdurchmesser groß genug, um eine hohe Bildqualität und eine hohe Auflösung von durch den Schaft aufgenommenen Bildern zu ermöglichen. Insbesondere kann dann im Inneren des Schaftes eine bildleitende Optik vorhanden sein, mit der von einem mittels der endoskopischen Sonde beobachteten Objekt ausgehendes Objektlicht zu einem im Handgriff angeordneten Bildsensor, der vorzugsweise ein HD-Sensor ist, geleitet wird. Zudem verbleibt im Schaft genügend Platz, damit Beleuchtungslicht, das von einer im Inneren des Handgriffs angeordneten Lichtquelle emittiert wird und mit dem das mittels der endoskopischen Sonde beobachtete Objekt beleuchtet wird, mit Hilfe eines durch das Innere des Schaftes geführten Lichtleiters zu dem beobachteten Objekt geführt werden kann.

Der Handgriff der erfindungsgemäßen endoskopischen Sonde kann einen Durchmesser von 18 bis 38 mm, insbesondere von 24 bis 32 mm aufweisen. Zudem kann der Griffabschnitt und das Vorderende zusammen eine Länge im Bereich von 100 bis 120 mm, insbesondere im Bereich von 105 bis 115 mm aufweisen. Durch diese Abmessungen ist er kompakt genug, damit die endoskopische Sonde zusammen mit einem Operationsmikroskop zum Einsatz kommen kann, ohne dass dabei ein wesentliches Risiko der Kollision des Handgriffs mit dem Operationsmikroskop besteht und andererseits voluminös genug ist, um einerseits die erforderlichen technischen Elemente, wie Lichtquelle und Sensor aufnehmen zu können und zudem aber auch einen Wärmespeicher, der verhindert, dass sich der Handgriff punktuell stark erhitzt und damit die Handhabung unkomfortabel oder im Extremfall unmöglich wäre. Zudem erfordert die sichere Handhabung eines Instruments mit der beschriebenen Geometrie ebenfalls einen Griff bzw. Handgriff, der gegebenen Abmessungen.

In einer vorteilhaften weiteren Ausgestaltung der erfindungsgemäßen endoskopischen Sonde weist der Griffabschnitt des Handgriffs in einer zur gemeinsamen Ebene der Schaftachse, der Griffabschnittsachse und der Hinterendenachse senkrechten Ebene einen Querschnitt auf, der eine zum Schnittpunkt einer gedachten Verlängerung der Schaftachse mit einer gedachten Verlängerung der Hinterendenachse weisende Querschnittsoberseite und eine von diesem Schnittpunkt weg weisenden Querschnittsunterseite besitzt. Dabei weist die Querschnittsoberseite einen zentralen, vorzugsweise gewölbten Abschnitt und zwei sich an den zentralen Abschnitt anschließende weniger stark konvex oder konkav gewölbte oder gerade Abschnitte auf, welche Ausschnitte aus den Schenkeln eines gedachten gleichschenkligen Dreiecks bilden, dessen Spitze mittig über der Querschnittsoberseite liegt. Das gleichschenklige Dreieck kann dabei insbesondere ein gleichseitiges Dreieck sein. Diese Ausgestaltung des Griffabschnitts der endoskopischen Sonde basiert auf der Erkenntnis, dass der Griffabschnitt in der Regel nicht mit der gesamten Hand umfasst wird, sondern häufig zur besseren feinmotorischen Handhabung ähnlich dem Bogen einer Geige mit den Fingerspitzen gefasst wird, oder aber in der Beuge zwischen Daumen und Zeigefinger liegend auf dem Mittelfinger balanciert und mit den Spitzen von Daumen und Zeigefinger geführt wird. Die geraden Abschnitte der Querschnittsoberseite bilden dabei vorteilhafte Griffflächen zum Greifen des Griffabschnitts mit der Daumenspitze auf der einen Seite und den Fingerspitzen von in der Regel zumindest dem Zeigefinger und ggf. dem Mittelfinger auf der anderen Seite. Mit Hilfe der geraden Abschnitte, die als Griffflächen bzw. Griffstrukturen im Griffabschnitt dienen, kann eine sichere Handhabung und eine ergonomische Führung der endoskopischen Sonde gewährleistet werden. Durch die Symmetrie in Form eines gleichschenkligen Dreiecks ist die endoskopische Sonde sowohl für die sichere Handhabung mit der linken Hand als auch für die sichere Handhabung mit der rechten Hand geeignet.

Die Querschnittsunterseite des Querschnitts des Griffabschnittes kann eine Wölbung aufweisen, wobei die Querschnittsunterseite dann einen zentralen Bereich mit einer zentralen Wölbung und sich an den zentralen Bereich anschließende Abschnitte mit einer im Vergleich zur zentralen Wölbung geringeren Wölbung umfasst. Insbesondere können die Abschnitte mit der im Vergleich zur zentralen Wölbung geringeren konvexen oder konkaven Wölbung auch frei von einer Wölbung sein und/oder parallel zueinander verlaufen. Durch die Verringerung der Wölbung und insbesondere, wenn die Abschnitte mit geringerer Wölbung ganz ohne Wölbung als parallele Abschnitte ausgebildet sind, können verhältnismäßig scharfe Kanten an den Rändern dieser Abschnitte ausgebildet werden, die zur sicheren Handhabbarkeit und Führung der endoskopischen Sonde beitragen können.

Die Querschnittsunterseite kann zudem eine Auflagefläche für den Mittelfinger aufweisen. Diese kann beispielsweise in Form eines flachen Abschnitts der Querschnittsunterseite oder in Form einer Einbuchtung oder Mulde in der Querschnittsunterseite realisiert sein.

Um die Sicht auf das distale Ende des Schaftes möglichst nicht zu behindern, ist es vorteilhaft, wenn der Schaft derart am Vorderende des Handgriffs angeordnet ist, dass das Vorderende senkrecht zur Ausdehnungsrichtung der Schaftachse einen maximalen Überstand über den Schaft aufweist, der so bemessen ist, dass der Winkel θ zwischen einer vom distalen Ende des Schaftes ausgehenden, in der Ebene der Schaftachse, der Hinterendenachse und der Griffabschnittsachse verlaufenden und das Vorderende des Handgriffs tangierenden gedachten Linie nicht mehr als 8 Grad, vorzugsweise nicht mehr als 5 Grad beträgt. Weiterhin besteht die Möglichkeit, dass die Achse des Vorderendes des Handgriffs nicht mit der Schaftachse zusammenfällt, sondern dass die beiden Achsen einen Winkel zueinander aufweisen, der derart gewählt ist, dass am distalen Ende des Schaftes der Überstand des Vorderendes ausgeglichen ist, so dass der Winkel θ weiter verringert werden kann und insbesondere Null Grad sein kann.

In der erfindungsgemäßen endoskopischen Sonde können der Schaft und der Handgriff einschließlich der Verbindung zwischen dem Schaft und dem Handgriff flüssigkeits- und gasdicht ausgeführt sein, und der Schaft und der Handgriff können aus einem autoklavierbaren Material bestehen. Durch diese Ausgestaltung können der Schaft und der Handgriff in einem Autoklav sterilisiert werden. Diese Ausgestaltung eignet sich insbesondere für kabellose endoskopische Sonden. Bei endoskopischen Sonden mit Kabel sind auch das Kabel und die Verbindung zwischen dem Kabel und dem Handgriff flüssigkeits- und gasdicht ausgeführt, und das Kabel besteht aus einem autoklavierbaren Material. Durch diese Ausgestaltung kann auch endoskopische Sonde mit Kabel in einem Autoklav sterilisiert werden. Durch die Sterilisierbarkeit entfällt die Notwendigkeit der Verwendung eines Drapes, so dass die ergonomische Handhabung auf jeden Fall erhalten bleibt.

Weitere Merkmale, Eigenschaften und Vorteile der vorliegenden Erfindung ergeben sich aus der nachfolgenden Beschreibung eines Ausführungsbeispiels unter Bezugnahme auf die beiliegenden Figuren.
- Figur 1: zeigt ein Ausführungsbeispiel für eine endoskopische Sonde mit einem Handgriff und einem Schaft.
- Figur 2: zeigt einen Schnitt durch den Schaft entlang der Linie II-II in Figur 1.
- Figur 3: zeigt einen Schnitt durch den Schaft entlang der Linie III-III in Figur 1.
- Figur 4: zeigt einen Schnitt durch den Handgriff entlang der Linie IV-IV in Figur 1.
- Figur 5: zeigt ein Anwendungsbeispiel für die endoskopische Sonde zusammen mit einem Operationsmikroskop.

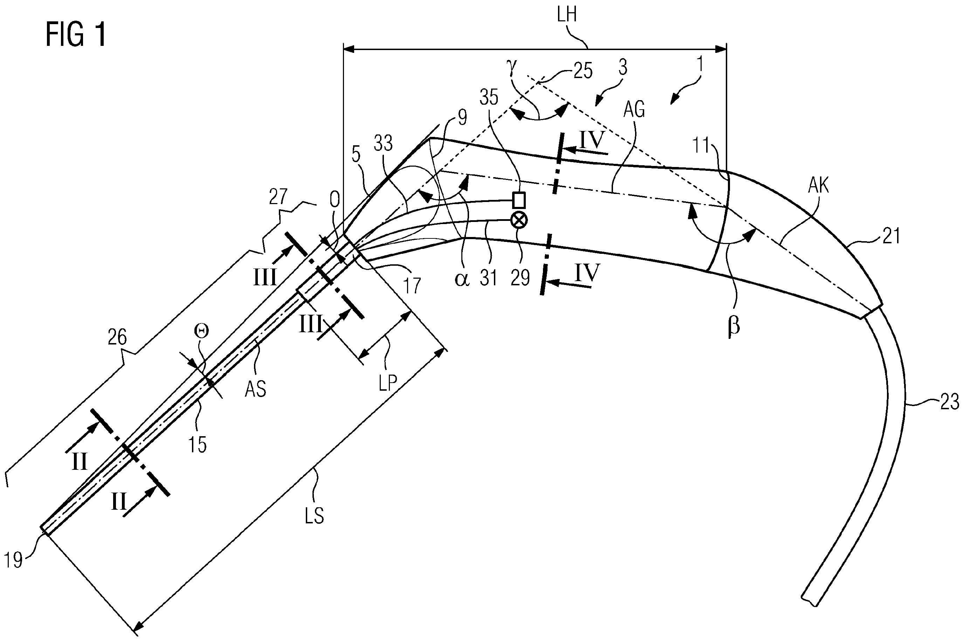

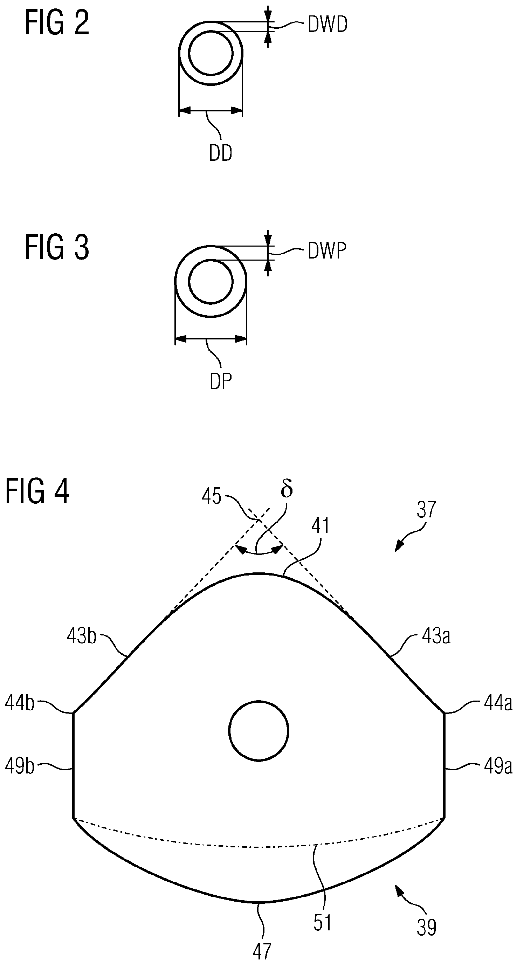

Ein Ausführungsbeispiel für eine erfindungsgemäße endoskopische Sonde wird nachfolgend mit Bezug auf die Figuren 1 bis 4 erläutert. Dabei zeigt Figur 1 eine Darstellung der endoskopischen Sonde mit einem Handgriff und einem Schaft und die Figuren 2 bis 4 Querschnitte durch den Schaft bzw. den Handgriff.

Die endoskopische Sonde umfasst im vorliegenden Ausführungsbeispiel einen Handgriff 1 mit einem Griffabschnitt 3, einem Vorderende 5 und einem Hinterende 21. Der Griffabschnitt 3 erstreckt sich entlang einer Griffabschnittsachse AG, wobei die Griffabschnittsachse die Mittelpunkte der beiden Endflächen 9, 11 des Griffabschnitts 3 miteinander verbindet. An die Endfläche 9 des Griffabschnitts schließt sich ein Vorderende 5 an, von dem aus sich der Schaft 15 entlang einer Schaftachse AS erstreckt. Der im vorliegenden Ausführungsbeispiel gerade Schaft 15 weist ein sich an das Vorderende 5 des Handgriffs 1 anschließendes proximales Ende 17 und ein vom Vorderende 5 des Handgriffs 1 entferntes distales Ende 19 auf. Seine Schaftachse AS schließt im vorliegenden Ausführungsbeispiel mit der Griffabschnittsachse AG einen Winkel α von 130 Grad ein. Es sei an dieser Stelle jedoch angemerkt, dass dieser Winkel α nicht notwendigerweise 130 Grad sein muss, sondern in einem Bereich von 100 bis 140 Grad, insbesondere in einem Bereich von 110 bis 130 Grad und vorzugsweise in einem Bereich von 115 bis 125 Grad liegen kann. Dieser Bereich, und insbesondere der engere der beiden Bereiche, hat sich als ergonomisch vorteilhaft herausgestellt.

An die andere Endfläche 11 des Griffabschnitts 3 schließt sich das Hinterende 21 des Handgriffs 1 an, aus dem im vorliegenden Ausführungsbeispiel ein Kabel 23 aus dem Handgriff 1 austritt. Das Hinterende 21 weist eine Hinterendenachse AK auf, wobei die Hinterendenachse AK durch den Mittelpunkt der sich an die Endfläche 11 des Griffabschnitts anschließenden Querschnittsfläche und durch den Mittelpunkt der Austrittsfläche des Kabels 23 verläuft. Das Kabel 23 tritt vorzugsweise in einer Richtung aus dem Hinterende 21 aus, die der Richtung der Hinterendenachse AK entspricht. Es kann grundsätzlich aber auch in einem kleinen Winkel zur Hinterendenachse AK austreten, wobei der kleine Winkel nicht mehr als maximal 20 Grad, vorzugsweise nicht mehr als 10 Grad aufweist.

Die Hinterendenachse AK schließt im vorliegenden Ausführungsbeispiel mit der Griffabschnittsachse AG einen Winkel β von 130 Grad sein. Es sei an dieser Stelle jedoch darauf hingewiesen, dass der Winkel β in anderen Ausführungsvarianten im Bereich zwischen 110 und 130 Grad, vorzugsweise im Bereich zwischen 115 und 125 Grad liegen kann. Der Bereich von 110 bis 130 Grad, und insbesondere der Bereich von 115 bis 125 Grad, hat sich dabei als ergonomisch vorteilhaft erwiesen.

Die Schaftachse AS, die Griffabschnittsachse AG und die Hinterendenachse AK liegen in einer gemeinsamen Ebene, wodurch sich gedachte Verlängerungen der Schaftachse AS und der Hinterendenachse AK in einem Schnittpunkt 25 schneiden. Die gedachten Verlängerungen der beiden Achsen sind in Fig. 1 gestrichelt eingezeichnet. Sie schließen im vorliegenden Ausführungsbeispiel einen Winkel γ von 80 Grad ein. Auch hier gilt jedoch, dass der Winkel γ nicht notwendigerweise 80 Grad sein muss, sondern in einem Bereich zwischen 30 und 90 Grad, beispielsweise 60 Grad liegen kann. Die genannten Bereiche für den Winkel γ ergeben sich aus den zuvor angegebenen Winkelbereichen für die Winkel α und β. Dadurch, dass alle drei Achsen AS, AG und AK in einer gemeinsamen Ebene liegen, kann die endoskopische Sonde so ausgebildet werden, dass sie sowohl mit der linken Hand als auch mit der rechten Hand gleichermaßen vorteilhaft geführt werden kann.

Im vorliegenden Ausführungsbeispiel weist der Griffabschnitt 3 zusammen mit dem Vorderende 5 eine Länge LH von 110 mm auf. Von diesem Wert kann jedoch auch abgewichen werden. Mögliche Längen des Griffabschnitts 3 einschließlich des Vorderendes 5 können im Bereich zwischen 100 und 120 mm liegen.

Der Schaft 15 besitzt im vorliegenden Ausführungsbeispiel vom proximalen Ende 17 bis zum distalen Ende 19 eine Länge LS von 120 mm. Auch hier ist anzumerken, dass in anderen Ausführungsvarianten der endoskopischen Sonde die Schaftlänge LS einen anderen Wert in einem Bereich von 50 bis 180 mm, insbesondere im Bereich von 90 bis 130 mm aufweisen kann. Der Außendurchmesser DD des Schaftes (siehe auch Figur 2, die einen Schnitt entlang der Line II-II aus Figur 1 zeigt) beträgt in einem distalen Abschnitt 26 des Schaftes 15 3,6 mm, und die Wanddicke DWD im distalen Abschnitt 26 beträgt 0,4 mm. In anderen Ausführungsvarianten der erfindungsgemäßen endoskopischen Sonde können der Außendurchmesser DD und die Wanddicke DWD im distalen Abschnitt 26 des Schaftes 15 auch andere Werte annehmen, wobei die Werte für den Außendurchmesser DD im Bereich zwischen 3,0 und 4,1, insbesondere im Bereich von 3,4 bis 3,8 mm, liegen und die Werte für die Wanddicke DWD im Bereich zwischen 0,4 und 0,5 mm, insbesondere im Bereich von 0,35 bis 0,45 mm, liegen. In einem zwischen dem distalen Abschnitt 26 und dem proximalen Ende 17 des Schaftes 15 gelegenen proximalen Abschnitt 27 (siehe auch Figur 3, die einen Schnitt entlang der Line III-III aus Figur 1 zeigt) weist der Schaft eine erhöhte Wandstärke DWP auf, die im vorliegenden Ausführungsbeispiel bei 1,4 mm liegt und in anderen Ausführungsvarianten der erfindungsgemäßen endoskopischen Sonde im Bereich von 1,3 bis 1,5 mm, insbesondere im Bereich von 1,35 bis 1,45 mm, liegen kann. Da der Innendurchmesser des Schaftes über die gesamte Länge des Schaftes gleich ist, ist der Außendurchmesser DP des Schaftes 15 im proximalen Abschnitt 27 entsprechend vergrößert. Der proximale Abschnitt erstreckt sich im vorliegenden Beispiel über eine Länge LP von 15 mm, er kann in anderen Ausführungsvarianten jedoch auch andere Werte im Bereich zwischen 10 und 20 mm, insbesondere im Bereich von 13 bis 17, mm annehmen. Durch die Verstärkung im proximalen Abschnitt wird die Stabilität des Schaftes erhöht. So lässt sich ein Schaft mit hoher Stabilität beig gleichzeitig geringem Durchmesser im distalen Abschnitt und großer Apertur realisieren.

Die genannten Durchmesser und Wandstärken des Schaftes 15 sind so gewählt, dass sie einerseits dem Schaft ermöglichen, in schmale Operationskanäle eingeführt zu werden, der Schaft andererseits aber eine ausreichende Stabilität aufweist, dass er bei der Handhabung nicht beschädigt wird. Zudem ist der Innendurchmesser des Schaftes groß genug, um einerseits eine Lichtleitfaser 31 hindurchführen zu können, mit der Licht einer im Handgriff 1 angeordneten Lichtquelle 29 zum distalen Ende 19 des Schaftes 15 geleitet werden kann, und andererseits von dem mittels der endoskopischen Sonde beobachteten Beobachtungsobjekt reflektiertes Licht über eine bildleitende Optik, die im vorliegenden Fall als geordnetes Faserbündel 33 ausgeführt ist, zu einem digitalen Bildsensor 35, beispielsweise einem CCD-Sensor oder einem CMOS-Sensor, geleitet werden kann. Alternativ besteht auch die Möglichkeit, die Optik in Form von Linsenkombinationen auszuführen. Insbesondere ermöglichen es die Abmessungen des Schaftes diesem, eine bildleitende Optik aufzunehmen, welche eine Aufnahme von HD-Videos mittels des digitalen Bildsensors 35 ermöglicht. Ebenso ist es möglich, den digitalen Bildsensor 35 an oder in der distalen Spitze des Schaftes 15 anzuordnen.

Um einem Anwender der endoskopischen Sonde ein ergonomisches Handhaben der Sonde zu ermöglichen, weist der Griffabschnitt 3 des Handgriffs 1 einen speziellen Querschnitt auf. Dieser Querschnitt ist in Figur 4 dargestellt, die einen Schnitt entlang der Linie IV-IV in Figur 1 zeigt. Der Querschnitt liegt in einer Ebene, die zur gemeinsamen Ebene der Schaftachse AS, der Griffabschnittsachse AG und der Hinterendenachse AK senkrecht verläuft. Der in Figur 4 gezeigte Querschnitt weist eine Querschnittsoberseite 37 und eine Querschnittsunterseite 39 auf. Die Querschnittsoberseite 37 bildet dabei im Griffabschnitt 3 diejenige Seite des Griffabschnittes, auf der der Schnittpunkt 25 zwischen den gedachten Verlängerungen der Schaftachse AS und der Hinterendenachse AK liegt.

In der erfindungsgemäßen endoskopischen Sonde weist die Querschnittsoberseite 37 einen gewölbten zentralen Abschnitt 41 und zwei sich daran anschließende im vorliegenden Ausführungsbeispiel leicht gewölbte Abschnitte 43a, 43b auf. Die Wölbungen können entlang der Griffabschnittsachse AG variieren. Die Tangenten an den leicht gewölbten Abschnitten 43a, 43b bilden dabei Ausschnitte aus den Schenkeln eines gedachten gleichschenkligen Dreiecks, dessen Spitze mittig über der Querschnittsoberseite 41 liegt. Der Winkel δ an der Spitze liegt dabei im Bereich zwischen 45 und 75 Grad und beträgt im vorliegenden Ausführungsbeispiel zumindest in einem Bereich des Griffabschnittes 60 Grad. Die von den leicht gewölbten Abschnitten 43a, 43b der Querschnittsoberseite gebildeten Flächen des Griffabschnitts 3 stellen Griffflächen zum Greifen des Griffabschnitts mit dem Daumen einerseits und der Spitze des Zeigefingers andererseits dar.

Die Querschnittsunterseite 39 weist im vorliegenden Ausführungsbeispiel einen zentralen Bereich 47 mit einer zentralen Wölbung auf, an denen sich zu beiden Seiten Abschnitte 49a, 49b anschließen, die im Vergleich zur zentralen Wölbung eine geringere Wölbung, also eine Wölbung mit einem größeren Krümmungsradius, aufweisen. Im vorliegenden Ausführungsbeispiel ist der Krümmungsradius sehr groß, so dass die Bereiche 49a, 49b gar keine Wölbung aufweisen. Die Bereiche 49a, 49b verlaufen dabei symmetrisch zur Symmetrieachse des gedachten gleichschenkligen Dreiecks der Querschnittsoberseite 37. Auf diese Weise können an den Verbindungen der geraden Abschnitte 49a, 49b der Querschnittsunterseite mit den geraden Abschnitten 43a, 43b der Querschnittsoberseite verhältnismäßig scharfe Kanten 44a, 44b entstehen, die eine taktile Markierung bilden können und so zur Sicherheit bei der Handhabung der endoskopischen Sonde beitragen.

Im vorliegenden Ausführungsbeispiel besteht die Möglichkeit, in der von der Querschnittsunterseite 39 gebildete Seite des Griffabschnitts 3 eine Anlagefläche für den Mittelfinger auszubilden, auf der der Mittelfinger ruhen kann. Diese Anlagefläche kann ein Bereich des Griffabschnitts 3, in dem der zentrale Bereich der Querschnittsunterseite 39 eine flachere Wölbung aufweist, sein, wie dies in Figur 4 durch die gestrichelte Linie 51 angedeutet ist.

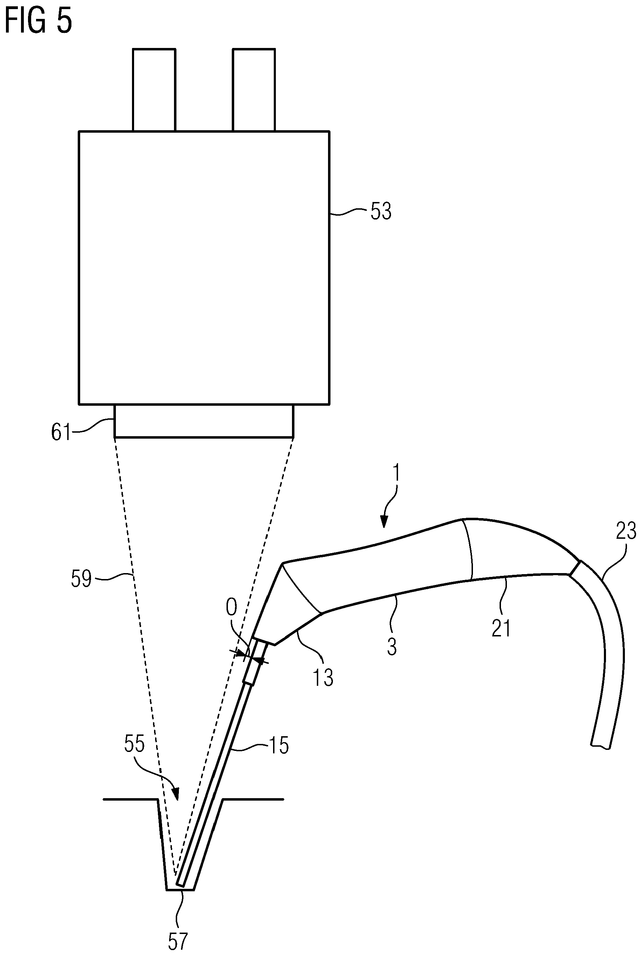

Die Verwendung einer erfindungsgemäßen endoskopischen Sonde zusammen mit einem Operationsmikroskop 53 ist in Figur 5 schematisch dargestellt. In dem in Figur 5 dargestellten Beispiel werden mit Hilfe des Schaftes 15 der endoskopischen Sonde Bilder vom Boden 57 eines Operationskanals 55 aufgenommen. Gleichzeitig wird der Operationskanal 55 während der Handhabung der endoskopischen Sonde mit dem Operationsmikroskop 53 betrachtet, um die Bewegung des Schaftes 15 im Operationskanal 55 zu überwachen. Hierbei ist es von Bedeutung, dass die endoskopische Sonde bei einer Bewegung innerhalb des Operationskanals 55, beispielsweise bei einer Drehung um die Schaftachse AS, beim Einführen in den Operationskanal 55 oder beim Herausziehen aus dem Operationskanal 55 nicht mit dem Operationsmikroskop 53 kollidiert. Eine solche Kollision kann durch die beschriebene abgewinkelte Geometrie des Schaftes 15, des Griffabschnittes 3 und des Hinterendes 21 erreicht werden. Das gegenüber dem Griffabschnitt 3 abgewinkelte Hinterende 21 ermöglicht es, das im vorliegenden Ausführungsbeispiel vom Hinterende 21 ausgehende Kabel 23 günstig über den Handrücken abzuführen.

Damit der Handgriff 1, und insbesondere das Vorderende 5, den mit dem Operationsmikroskop 53 beobachteten Beobachtungsbereich möglichst wenig verdeckt, weist das Vorderende 5 in einer Richtung senkrecht zur Schaftachse AS einen Überstand O auf, der so bemessen ist, dass der Winkel θ (vgl. Figur 1) zwischen einer vom distalen Ende 19 des Schaftes 15 ausgehenden, in der Ebene der Schaftachse AS, der Hinterendenachse AK und der Griffabschnittsachse AG verlaufenden und das Vorderende 5 des Handgriffs tangierenden gedachten Linie nicht mehr als 8 Grad, vorzugsweise nicht mehr als 5 Grad beträgt. Hierzu kann der Überstand O im vorliegenden Ausführungsbeispiel maximal einem Zehntel, insbesondere maximal einem Fünfzehntel und vorzugsweise maximal einem Zwanzigstel der Schaftlänge LS entsprechen. Im vorliegenden Ausführungsbeispiel beträgt der Überstand 5 mm bei einer Schaftlänge von 120 mm, was einem Vierundzwanzigstel der Schaftlänge entspricht. Der Winkel θ beträgt 3 Grad. Wie in Figur 5 angedeutet ist, kann dadurch erreicht werden, dass der zum Hauptobjektiv 61 führende Öffnungskegel 51 des Beobachtungsstrahlengangs nicht oder höchstens geringfügig durch die endoskopische Sonde abgeschattet wird.

Zudem besteht auch die Möglichkeit, die Richtung der Schaftachse AS so zu wählen, dass das distale Ende 19 gegenüber dem Punkt, an dem das proximale Ende 17 am Vorderende 5 des Handgriffs 1 ansetzt, um den Überstand O in Radialrichtung versetzt ist, so dass das Vorderende 5 die Sicht auf das distale Ende des Schaftes 15 noch weniger behindert.

Im vorliegenden Ausführungsbeispiel sind der Schaft 15 und der Handgriff 1 einschließlich der Verbindung zwischen dem Schaft 15 und Handgriff 1 flüssigkeits- und gasdicht ausgeführt. Zudem sind der Schaft 15 und der Handgriff 1 aus einem Material hergestellt, das in einem Autoklaven sterilisiert werden kann. Dadurch, dass die endoskopische Sonde flüssigkeits- und gasdicht ausgestaltet ist, sind die im Inneren des Handgriffs und des Schaftes angeordneten Komponenten während der Behandlung im Autoklaven geschützt, so dass sie durch die Behandlung nicht beeinträchtigt werden. Ein drapefreies Arbeiten wist durch möglich.

Im beschriebenen Ausführungsbeispiel betrugen der Winkel α 130 Grad, der Winkel β 130 Grand und der Winkel γ 80 Grad. In einem anderen Ausführungsbeispiel betragen der Winkel α 120 Grad, der Winkel β 120 Grand und der Winkel γ 60 Grad.

Die vorliegende Erfindung wurde zu Erläuterungszwecken anhand eines Ausführungsbeispiels detailliert beschrieben. Ein Fachmann erkennt jedoch, dass von diesem Ausführungsbeispiel abgewichen werden kann. Bspw. kann der im Ausführungsbeispiel gerade Schaft auch leicht gebogen sein, wobei zum Ermitteln der Winkel zu den anderen Achsen dann die Orientierung der Schaftachse in ihrem unmittelbar an den Schaftabschnitt des Handgriffes anschließenden Abschnitt heranzuziehen ist. Weitere Abweisungen vom Ausführungsbeispiel sind möglich, wie an verschiedenen Stellen des Ausführungsbeispiels bereits ausgeführt worden ist. Weiterhin kann die Endoskopische Sonde kabellos ausgeführt sein, wobei die Datenübertragung dann bspw. über WLAN, Bluetooth oder Infrarotübertragung erfolgen kann. Im Hinterende ist dann bspw. eine Antenne für die drahtlose Übertragung oder ein Akkumulator, der die für die Übertragung benötigte Energie liefert, vorhanden. Ein sterilisierbares Kabel entfällt dann. Die Erfindung soll daher nicht auf das kabelgebundene Ausführungsbeispiel beschränkt sein, sondern lediglich auf eine endoskopische Sonde, wie sie in den beigefügten Ansprüchen beansprucht ist.

### Bezugszeichenliste

- 1: Handgriff
- 3: Griffabschnitt
- 5: Vorderende
- 7: Hinterende
- 9: Endfläche
- 11: Endfläche
- 15: Schaft
- 17: proximales Ende
- 19: distales Ende
- 21: Hinterende
- 23: Kabel
- 25: Schnittpunkt
- 26: distaler Abschnitt
- 27: proximaler Abschnitt
- 29: Lichtquelle
- 31: Lichtleitfaser
- 33: Faserbündel
- 35: digitaler Bildsensor
- 37: Querschnittsoberseite
- 39: Querschnittsunterseite
- 41: gewölbter zentraler Abschnitt
- 43a, b: gerader Abschnitt
- 44a, 44b: Kante
- 45: Dreiecksspitze
- 47: zentraler Bereich
- 49a, b: gerader Abschnitt
- 51: flachere Unterseite
- 53: Operationsmikroskop
- 55: Operationskanal
- 57: Boden
- 59: Öffnungskegel
- 61: Hauptobjektiv
- AG: Griffabschnittsachse
- AK: Hinterendenachse
- AS: Schaftachse
- DD: Außendurchmesser
- DP: Außendurchmesser
- DWD: Wandstärke
- DWP: Wandstärke
- LS: Schaftlänge
- LP: Läge proximaler Abschnitt

## Patentansprüche

1. Endoskopische Sonde umfassend:
- einen Handgriff (1) mit einem Vorderende (5), einem Hinterende (21) mit einer Hinterendenachse (AK) und einem sich zwischen dem Vorderende (5) und dem Hinterende (21) erstreckenden geraden Griffabschnitt (3) mit einer Griffabschnittsachse (AG);
- einen sich vom Vorderende (5) des Handgriffs (1) aus erstreckenden Schaft (15) mit einer Schaftachse (AS),
wobei
- die Schaftachse (AS), die Hinterendenachse (AK) und die Griffabschnittsachse (AG) innerhalb einer gemeinsamen Ebene liegen,
- die Schaftachse (AS) mit der Griffabschnittsachse (AG) einen Winkel (α) im Bereich von 100 bis 140 Grad einschließt,
- die Griffabschnittsachse (AG) mit der Hinterendenachse (AK) einen Winkel (β) im Bereich von 110 bis 130 Grad einschließt,
- die Schaftachse (AS) mit der Hinterendenachse (AK) einen Winkel (γ) im Bereich von 30 bis 90 Grad einschließt. und

2. Endoskopische Sonde nach Anspruch 1, in der der Winkel, den die Schaftachse (AS) mit der Griffabschnittsachse (AG) einschließt, denselben Betrag besitzt wie der Winkel, den die Griffabschnittsachse (AG) mit der Hinterendenachse einschließt.

3. Endoskopische Sonde nach Anspruch 1 oder Anspruch 2, in der der Schaft (15) eine Länge im Bereich von 110 bis 130 mm aufweist.

4. Endoskopische Sonde nach einem der Ansprüche 1 bis 3, in der der Schaft (15) einen Durchmesser im Bereich von 3,0 bis 4,1 mm aufweist.

5. Endoskopische Sonde nach einem der Ansprüche 1 bis 4, in der der Schaft (15) zumindest in einem distalen Abschnitt eine Wandstärke im Bereich von 0,3 bis 0,5 mm aufweist.

6. Endoskopische Sonde nach einem der Ansprüche 1 bis 5, in der der Schaft (15) einen sich an das Vorderende (5) des Handgriffs (1) anschließenden proximalen Abschnitt (27) mit einer Wandstärke im Bereich von 1,3 bis 1,5 mm aufweist.

7. Endoskopische Sonde nach Anspruch 6, in der der proximale Abschnitt (27) eine Länge im Bereich von 10 bis 20 mm aufweist.

8. Endoskopische Sonde nach einem der Ansprüche 1 bis 7, in der der Griffabschnitt (3) einen Durchmesser im Bereich von 18 bis 38 mm aufweist.

9. Endoskopische Sonde nach einem der Ansprüche 1 bis 8, in der der Griffabschnitt (3) und das Vorderende (5) zusammen eine Länge (LH) im Bereich von 100 bis 120 mm aufweist.

10. Endoskopische Sonde nach einem der Ansprüche 1 bis 9, in der der Griffabschnitt (3) in einer zur gemeinsamen Ebene der Schaftachse (AS), der Griffabschnittsachse (AG) und der Hinterendenachse (AK) senkrechten Ebene einen Querschnitt mit einer Querschnittsoberseite (37), welche die zum Schnittpunkt einer gedachten Verlängerung der Schaftachse (AS) mit einer gedachten Verlängerung der Hinterendenachse (AK) weisende Seite des Handgriffs (1) bildendet, und einer von der Querschnittsoberseite (37) weg weisenden Querschnittsunterseite (39) aufweist, wobei die Querschnittsoberseite (37) einen zentralen Abschnitt und zwei sich an den zentralen Abschnitt anschließende gerade Abschnitte aufweist, welche Ausschnitte aus den Schenkeln eines gedachten gleichschenkligen Dreiecks bilden, dessen Spitze mittig über der Querschnittsoberseite (37) liegt.

11. Endoskopische Sonde nach Anspruch 10, in der die Querschnittsunterseite (39) eine Wölbung aufweist, wobei die Querschnittsunterseite (39) einen zentralen Bereich mit einer zentralen Wölbung und sich an den zentralen Bereich anschließende Abschnitte mit einer im Vergleich zur zentralen Wölbung geringeren Wölbung umfasst.

12. Endoskopische Sonde nach Anspruch 10 oder Anspruch 11, in der die von der Querschnittsunterseite (37) gebildete Seite des Griffabschnitts (3) eine Anlagefläche für den Mittelfinger aufweist.

13. Endoskopische Sonde nach einem der vorangehenden Ansprüche, in der der Schaft (15) derart an dem Vorderende (5) des Handgriffs angeordnet ist, dass das Vorderende (5) senkrecht zur Ausdehnungsrichtung der Schaftachse (AS) einen maximalen Überstand über den Schaft (15) aufweist, der so bemessen ist, dass der Winkel θ zwischen einer vom distalen Ende (19) des Schaftes (15) ausgehenden, in der Ebene der Schaftachse (AS), der Hinterendenachse (AK) und der Griffabschnittsachse (AG) verlaufenden und das Vorderende (5) des Handgriffs (1) tangierenden gedachten Linie nicht mehr als 8 Grad beträgt..

14. Endoskopische Sonde nach Anspruch 13, in der das Vorderende (5) eine Vorderendenachse aufweist, die in einem Winkel zur Schaftachse (AS) verläuft, wobei der Winkel so gewählt ist, dass am distalen Ende (19) des Schaftes (15) der Überstand des Vorderendes (5) ausgeglichen ist.

15. Endoskopische Sonde nach einem der Ansprüche 1 bis 14, in der das Hinterende (21) ein Kabelabgangsende mit einem austretenden Kabel (23) ist, wobei das Kabel (23) in einem Winkel von nicht mehr als 20 Grad zur Hinterendenachse (AK) aus dem Kabelabgangsende (21) austritt.
